# FASCICULE DE BREVET EUROPEEN

(11) **EP 1 181 029 B1**
(45) Date de publication et mention de la délivrance du brevet: **22.01.2003**
(21) Numéro de dépôt: 00936993.5
(22) Date de dépôt: 30.05.2000
(51) Int. Cl.: A61K 35/78, A61P 17/02

(54) **BAUME TRAITANT NOTAMMENT POUR BRULURES SUPERFICIELLES ET ROUGEURS IRRITATIVES**
BEHANDLUNGSBALSAM, INSBESONDERE FÜR OBERFLÄCHLICHEN VERBRENNUNGEN UND IRRITIERENDEN RÖTUNGEN
BALSAM FOR THE TREATMENT OF SURFACE BURNS AND CHAFING RASHES

(30) Priorité: 01.06.1999 FR 9906859
(43) Date de publication de la demande: 27.02.2002
(73) Titulaire: Kyrn S.A., 06000 Nice (FR)
(72) Inventeur: PIETRI, Christine Of. Méditer. Bre. d'Inv. Marques, 06000 Nice (FR); VOLLE, Isabelle Office Méd. Brev. d'Inv. Marques, 06000 Nice (FR)
(74) Mandataire: Hautier, Jean-Louis
(86) Numéro de dépôt international: FR0001498
(87) Numéro de publication internationale: WO00072859

(56) Documents cités:
- EP-A- 0 258 481
- EP-A- 0 391 780
- FR-A- 2 750 331

## Description

L'invention a pour objet un baume traitant pour brûlures superficielles et rougeurs irritatives.

Les gels, baumes ou autres compositions ont plusieurs fonctions pour agir sur les brûlures superficielles, une fonction mécanique qui crée une barrière de protection.

L'essentiel de ces produits tendent à protéger par une barrière de protection la partie à traiter.

Cette barrière mécanique a pour effet secondaire de ne pas favoriser l'oxygénation.

Il existe quelques produits sur le marché actuellement :
BEPANTHENE (marque déposée) 5 % Onguent :
Forme topique d'administration de l'acide panthoténique précurseur du Coenzyme A et point de départ de la synthèse des acides gras, comme l'acide palmique, du cholestérol et des prostaglandines. L'acide palmitique est à l'origine de l'une des familles des acides gras polyinsaturés, qui joue un rôle régulateur important dans la sécheresse cutanée et ses manifestations d'irritations, sans action anti-inflammatoire.
BRULEX (marque déposée)
Pommade de traitement des brûlures superficielles à base de phénazone (antipyrine, anti-inflammatoire et antalgique), oxyde zinc (cicatrisant, anti-irritant), salicylate de Na (anti-inflammatoire) et baume du Pérou (anti-inflammatoire). L'efficacité anti-inflammatoire est certaine mais on note la présence de produits sensibilisants, comme le baume du Pérou et le Phénol, et potentiellement neurotoxiques.
BIAFINE (marque déposée TROLAMINE)
Emulsion (huile dans eau) à base de tromaline (Triethanolamine), base faible, et d'alginate de Triethanolamine de sodium dont les rôles sur la physiologie cutanée ne sont pas déterminants.
La valeur anti-erythémateuse et anti-inflammatoire de la préparation peut être due à la présence de perhydrosqualène, agent anti-irritant et fortement hydratant et à la présence d'huile d'avocat riche en insaponifiable à activité adoucissante.
Le reste des composants est tout à fait classique pour une émulsion savon standard.
Ces composants sont dénués de toute activité cutanée et le sens huile dans l'eau de l'émulsion ne permet pas d'envisager un important effet occlusif isolant et réhydratant.

L'état de la technique peut être défini également par les brevets suivants :
- *FR-2.454.303 : l'invention concerne une composition pharmaceutique à base de povidone iodée et de sucre. La composition selon l'invention comprend des mélanges de : un agent antifongique antibactérien, tel que la povidone iodée, du sucre et un support approprié. Ces mélanges sont pratiquement non allergènes, ont d'excellentes propriétés de cicatrisation lorsqu'ils sont appliqués sur des brûlures ou des plaies ouvertes, et servent de barrière efficace contre le développement d'un tissu neuf dans un pansement de gaze ou similaire. EP-A-0.391.780 : Composition anti-démangeaisons caractérisée en ce qu'elle renferme, en tant que composant actif, au moins un glycérol oxydo ester ayant été préparé par saturation en oxygène et exposition intensive et contrôlée aux ultravoliolets, de façon à présenter un taux de peroxydation compris entre 30 et 300 exprimé en milli-équivalents et une teneur en glycérides oxydés comprise entre 5 et 40, la définition du principe actif en spectrophotométrie UV étant de 1 à 6 pour le facteur E 270 et de 8 à 60 pour le facteur E 232. L'invention s'applique aux composition notamment aux lotions capillaires anti-démangeaisons.*
   Ce brevet décrit une composition pour une toute autre application (anti-démangeaison) que la présente invention et de plus la combinaison principale des composants n'est pas décrite.
   *FR-A-2.750.331 : L'invention concerne une composition cosmétique comprenant un ou plusieurs corps gras peroxydés caractérisé en ce qu'elle comporte, en outre, un ou plusieurs corps gras non peroxydés en quantité comprise entre 10 et 50%. L'invention concerne en outre l'application de la composition cosmétique notamment à la prévention des escarres, des troubles de la micro-circulation, des états pelliculaires.*
   Ce brevet décrit une composition pour une toute autre application, prévention des escarres, des troubles de la micro-circulation, des états pelliculaires, que la présente invention et de plus la combinaison principale des composants n'est pas décrite.
- *EP-A-0.258.481 : Ce brevet décrit une composition pharmaceutique pour le traitement des maladies de la peau. Il utilise l'α-tocopherol connu pour ses effets anti-radiculaires. L'application concerne la pathologie infectieuse.*
*La présente invention concerne un traumatisme de la peau ou une irritation, de plus la combinaison principale n'est pas décrite.*

L'invention tend à éviter tous ces problèmes notamment en combinant la présence d'une action mécanique faisant office de barrière et l'action d'un agent favorisant l'oxygénation des cellules.

Baume traitant pour les brûlures du type utilisant :
- un décongestionnant et calmant,
- un agent de reconstruction de l'épiderme,
- un agent adoucissant,
- un protecteur et/ou régénérateur membranaire,
- et autres excipients
caractérisé par le fait qu'il comprend une huile peroxydée dont la fonction est à la fois occlusive, anti-inflammatoire, antalgique et qui agit comme agent favorisant l'oxygénation des cellules, ledit baume comprend également du tocopheryl quinone qui a une action vasodilatatrice et oxygénatrice.

Le pourcentage d'huile peroxydée est de 50 % à 90 %.

Le pourcentage de tocopheryl quinone est de 0, 01 % à 3 %.

L'huile péroxydée est préférentiellement de l'huile de maïs.

L'huile péroxydée est l'huile de soja.

L'huile péroxydée est l'huile de noix.

L'huile péroxydée est l'huile de pépin de raisin.

L'huile péroxydée est l'huile de tournesol.

Selon un mode de réalisation, la composition du baume est la suivante :
une huile neutre occlusive
un agent de gélification des corps gras
un extrait végétal huileux décongestionnant et calmant
une huile riche en acide gamma linolénique, agent de reconstruction de l'épiderme
Acétate de vitamine E : protecteur membranaire
Acétate de vitamine A : régénération des ephitéliums stratifiés
Carotène huileux : provitamine A
Huile peroxydée : 50 % à 90 %
Tocopheryl quinone : 0,01 % à 3 %.

L'huile neutre occlusive est l'huile de vaseline.

L'extrait végétal huileux est du végétal huileux Calendula

L'agent adoucissant est la cire d'abeille

L'huile riche en acide gamma linolénique est l'huile de bourrache.

Le baume selon l'invention s'applique notamment aux brûlures superficielles ou aux rougeurs irritatives.

Ces brûlures peuvent être dues au soleil ou à des traitements de la peau comme par exemple des traitements au laser.

## Revendications

1. Baume traitant notamment pour les brûlures du type utilisant :
- un décongestionnant et calmant,
- un agent de reconstruction de l'épiderme,
- un agent adoucissant,
- un protecteur et/ou régénérateur membranaire,
**caractérisé par le fait**
**qu'**il comprend, en combinaison :
- une huile peroxydée combinant une action mécanique par formation d'une barrière physique occlusive de la partie du corps à traiter et une action chimique anti-inflammatoire, antalgique et oxygénant des cellules ;
- du tocopheryl quinone pour une action de vasodilatation.

2. Baume traitant selon la revendication 1 **caractérisé par le fait**
**que** l'huile peroxydée est l'huile de mais.

3. Baume traitant selon la revendication 1 **caractérisé par le fait**
**que** l'huile peroxydée est l'huile de soja.

4. Baume traitant selon la revendication 1 **caractérisé par le fait**
**que** l'huile peroxydée est l'huile de noix.

5. Baume traitant selon la revendication 1 **caractérisé par le fait**
**que** l'huile peroxydée est l'huile de pépin de raisin.

6. Baume traitant selon la revendication 1 **caractérisé par le fait**
**que** l'huile peroxydée est l'huile de tournesol.

7. Baume traitant selon la revendication 1 **caractérisé par le fait**
**que** le pourcentage d'huile peroxydée est de 50 % à 90 %.

8. Baume traitant selon la revendication 1 **caractérisé par le fait**
**que** le pourcentage de tocopheryl quinone est de 0,01 % à 3 %.

9. Baume traitant selon la revendication 1 **caractérisé par le fait**
**que** la composition du baume est la suivante :
- Huile neutre occlusive
- Agent de gélification des corps gras
- Extrait végétal huileux : décongestionnant et calmant
- Huile riche en acide gamma linolénique, agent de reconstruction de l'épiderme
- Agent adoucissant
- Acétate de vitamine E : protecteur membranaire
- Acétate de vitamine A : régénération des ephitéliums stratifiés
- Carotène huileux : provitamine A
- Huile peroxydée : 50 % à 90 %
- Tocopheryl quinone : 0,01 % à 3 %.

10. Baume traitant selon la revendication 9 **caractérisé par le fait**
**que** l'extrait végétal huileux est du végétal huileux calendula.

11. Baume traitant selon l'une quelconque des revendications 1 à 10 **caractérisé par le fait**
**que** l'agent adoucissant est la cire d'abeille.

12. Baume traitant selon l'une quelconque des revendications 1 à 11 **caractérisé par le fait**
**que** l'huile riche en acide gamma linolénique est l'huile de bourrache.

13. Utilisation, pour la fabrication d'un baume de traitement des brûlures, d'une huile peroxydée comme agent de formation d'une barrière physique occlusive de la partie du corps à traiter et de tocopheryl quinone comme agent d'oxygénation des cellules.

## Patentansprüche

1. Salbe, insbesondere zur Behandlung von Verbrennungen,
- mit abschwellender und schmerzlindernder Wirkung,
- mit einer die Heilung unterstützenden Substanz,
- mit einer reizmildernden Substanz
- mit einem Membranschutz- oder Membranregeneriermittel,
**gekennzeichnet dadurch,**
**daß** sie eine Kombination darstellt, aus
- einem Peroxyd-Öl, das physikalisch eine dichte Deckschicht für die zu behandelnden Hautpartien bildet und chemisch eine entzündungshemmende und schmerzlindernde Wirkung besitzt und die Sauerstoffzufuhr der Zellen fördert,
- und Tocopheryl-chinon wegen dessen gefäßerweiternder Wirkung.

2. Salbe gemäß Anspruch 1, **gekennzeichnet dadurch,**
**daß** das Peroxyd-Öl Maisöl ist.

3. Salbe gemäß Anspruch 1, **gekennzeichnet dadurch,**
**daß** das Peroxyd-Öl Sojaöl ist.

4. Salbe gemäß Anspruch 1, **gekennzeichnet dadurch,**
**daß** das Peroxyd-Öl Nussöl ist.

5. Salbe gemäß Anspruch 1, **gekennzeichnet dadurch,**
**daß** das Peroxyd-Öl Traubenkernöl ist.

6. Salbe gemäß Anspruch 1, **gekennzeichnet dadurch,**
**daß** das Peroxyd-Öl Sonnenblumenöl ist.

7. Salbe gemäß Anspruch 1, **gekennzeichnet dadurch,**
**daß** der Anteil an Peroxyd-Öl zwischen 50 % und 90 % liegt.

8. Salbe gemäß Anspruch 1, **gekennzeichnet dadurch, daß**
der Anteil des Tocopheryl-chinons zwischen 0,01 % und 3 % liegt.

9. Salbe gemäß Anspruch 1, **gekennzeichnet dadurch, daß** sie folgende
Zusammensetzung hat:
- neutrales Öl, das eine mechanisch dichte Schicht bildet
- Geliermittel für Fette
- Ölhaltige Pflanzenauszüge mit abschwellender und schmerzlindernder Wirkung
- Öl mit hohem Anteil an Gamma-Linolsäure mit regenerierender Wirkung auf die Epidermis
- Reizlindernde Mittel
- Vitamin E-Azetat: Membranschutz
- Vitamin A-Acetat: zur Regenerierung des mehrschichtigen Epitels
- Ölhaltiges Karotin: Provitamin A
- Peroxyd-Öl: 50 % bis 90 %
- Tocopheryl-chinon: 0,01 % bis 3 %

10. Salbe gemäß Anspruch 9, **gekennzeichnet dadurch,**
**daß** der ölhaltige Pflanzenauszug aus der Ölpflanze Calendula stammt.

11. Salbe gemäß einem der vorstehenden Ansprüche, **gekennzeichnet dadurch,**
**daß** das reizlindernde Mittel Bienenwachs ist.

12. Salbe gemäß einem der vorstehenden Ansprüche 1 bis 11, **gekennzeichnet dadurch,**
**daß** das an Gamma-Linolsäure reiche Öl Borretsch-Öl ist.

13. Verwendung eines Peroxyd-Öls zur Herstellung einer Brandsalbe zur Bildung einer physikalisch dichten Deckschicht auf der zu behandelnden Hautpartie und von Tocopheryl-chinon zur Sauerstoffzufuhr für die Zellen.

## Claims

1. Balm notably for the treatment of burns that uses
- a decongestant and pain reliever,
- an agent for rebuilding the epidermis,
- a softening agent,
- membranal protector and/or regenerator, **characterized in that**
it includes, in combination:
- a peroxidized oil combining a mechanical action by the formation of an occlusive physical barrier for the part of the body to be treated and a drug with anti-inflammatory, analgesic chemical action and oxygenating the cells;
- tocopheryl quinone for a vasodilatation action.

2. Treatment balm according to claim 1 **characterized in that** the peroxidized oil is corn oil.

3. Treatment balm according to claim 1 **characterized in that** the peroxidized oil is soya bean oil.

4. Treatment balm according to claim 1 **characterized in that** the peroxidized oil is walnut oil.

5. Treatment balm according to claim 1 **characterized in that** the peroxidized oil is grape seed oil.

6. Treatment balm according to claim 1 **characterized in that** the peroxidized oil is sunflower oil.

7. Treatment balm according to claim 1 **characterized in that** the percentage of peroxidized oil is between 50% and 90%.

8. Treatment balm according to claim 1 **characterized in that** the percentage of tocopheryl quinone is between 0.01% and 3%.

9. Treatment balm according to claim 1 **characterized in that** the balm composition is as follows:
- Occlusive neutral oil
- Agent for gelling greasy substances
- Decongestant and pain relieving plant oil extract
- Gamma linoleic acid-rich oil, agent for rebuilding the epidermis
- Softening agent
- Vitamin E acetate: membranal protector
- Vitamin A acetate regeneration of stratified ephitelia
- Oily carotene: provitamin A
- Peroxidized oil : 50% to 90%
- Tocopheryl quinone: 0.01% to 3%.

10. Treatment balm according to claim 9 **characterized in that** the vegetable oil extract is oily plant calendula officinalis.

11. Treatment balm according to any one of claims 1 to 10 **characterized in that**
the softening agent is beeswax.

12. Treatment balm according to any one of claims 1 to 11 **characterized in that**
the oil rich in linoleic gamma acid is the oil of borago officinalis.

13. Use, for the manufacture of a balm for the treatment of burns, of an peroxidized oil as agent to form an occlusive physical barrier for the part of the body to be treated, and of tocopheryl quinone as the cell oxygenating agent.
